# EUROPÄISCHE PATENTSCHRIFT

(11) **EP 1 229 851 B1**
(45) Veröffentlichungstag und Bekanntmachung des Hinweises auf die Patenterteilung: **31.03.2004**
(21) Anmeldenummer: 00979580.8
(22) Anmeldetag: 16.11.2000
(51) Int. Cl.: A61B 19/00

(54) **BILDAUFNAHMEVORRICHTUNG FÜR DEN EINSATZ IM STERILBEREICH ZUR VISUELLEN ÜBERWACHUNG UND DOKUMENTATION EINER CHIRURGISCHEN OPERATION**
IMAGE RECORDING DEVICE TO BE USED IN A STERILE AREA FOR VISUALLY MONITORING AND DOCUMENTING A SURGICAL INTERVENTION
DISPOSITIF DE PRISE D'IMAGES DESTINE A ETRE UTILISE DANS UNE ZONE STERILE POUR LA SURVEILLANCE ET LA DOCUMENTATION D'UNE OPERATION CHIRURGICALE

(30) Priorität: 16.11.1999 DE 19955041
(43) Veröffentlichungstag der Anmeldung: 14.08.2002
(73) Patentinhaber: Lemke, Norbert, 82199 Gröbenzell (DE); Lemke, Rosemarie, 82199 Gröbenzell (DE); Lemke, Thilo, 81371 München (DE)
(72) Erfinder: Lemke, Norbert, 82199 Gröbenzell (DE); Lemke, Rosemarie, 82199 Gröbenzell (DE); Lemke, Thilo, 81371 München (DE)
(74) Vertreter: Rösler, Uwe, Dipl.-Phys.
(86) Internationale Anmeldenummer: PCT/EP2000/011367
(87) Internationale Veröffentlichungsnummer: WO 2001/035848

(56) Entgegenhaltungen:
- EP-A- 0 293 760
- DE-A- 4 142 954
- DE-A- 19 521 060
- GB-A- 2 289 001
- US-A- 4 854 301
- US-A- 5 495 286
- US-A- 5 697 891

## Beschreibung

### Technisches Gebiet

Die Erfindung bezieht sich auf eine Bildaufnahmevorrichtung für den Einsatz im Sterilbereich zur visuellen Überwachung und Dokumentation von einer, am menschlichen oder tierischen Körper durchzuführenden chirurgischen Operation innerhalb eines bestimmten Operationsbereiches, mit einer Kameraeinheit und einer, mit dieser gekoppelten bildaufnehmenden Optikeinheit, die zusammen mit der Kameraeinheit extrakorporal derart positionierbar ist, dass die Optikeinheit einen Aufnahmebereich definiert, der zumindest den Operationsbereich enthält.

### Stand der Technik

Aufzeichnungssysteme der vorstehend genannten Gattung dienen zur Bildaufzeichnung von chirurgischen Eingriffen, vorzugsweise am menschlichen Körper, und finden aus einer Vielzahl von Gründen einen zunehmenden Einsatz. So ist es insbesondere für Studien- und Fortbildungszwecke vorteilhaft, wenn anhand einer Videoaufzeichnung die gesamte Operationsdurchführung auch nicht unmittelbar an der Operation beteiligten Personen optisch vermittelt werden kann.

Auch dienen derartige Bildaufzeichnungen der Nachweisbarkeit bestimmter chirurgischer Tätigkeiten, die heutzutage zunehmend öfter von Seiten der Krankenkassen eingefordert werden. Schließlich können auch Bildaufnahmen in digitaler Form vom Operationsgeschehen im Wege modernster Kommunikationstechniken ausgetauscht werden, sodass Experten weltweit via Internet bei der Durchführung komplizierter Operationen zusammengerufen werden können, die durch die optische Inbetrachtnahme des aktuellen Operationsgeschehens unterstützend zur Seite stehen können, Stichwort Telemedizin.

Zur Bildaufzeichnung des gesamten Operationsgeschehens sind Videokamerasysteme bekannt, die im Umfeld zum Operationstisch positioniert werden müssen. Neben dem Problem der nicht vollständigen Sterilisierbarkeit derartiger Videokamerasysteme können die Videokameras nicht in unmittelbarer Nähe zum Operationsbereich positioniert werden, zumal sie auf Grund ihrer Größe dem Operationsgeschehen nur hinderlich im Wege stehen würden. Aus diesem Grunde sind Aufnahmen mit herkömmlichen Videokameras lediglich aus der Ferne mit langbrennweitigen Objektiven durchzuführen, was jedoch mit einer Reihe von Nachteilen behaftet ist. So ist es zum einen schwierig, auf Grund der notwendigen langbrennweitigen Objektive stets für scharfe Abbildungen zu sorgen, zumal der Fokusbereich nur in einem räumlich sehr eng begrenzten Abstand zum Objektiv liegt. Ferner kommt es häufig vor, dass der freie Sichtbereich zwischen Videokamera und Operationsfeld durch die an der Operation beteiligten Personen verdeckt ist.

Zur Vermeidung dieses Nachteils sind optische Aufzeichnungssysteme bekannt, die in den Beleuchtungssystemen integriert sind, die für eine optimale Ausleuchtung des Operationsbereiches vorgesehen sind. Operationsleuchten werden in aller Regel derart über dem Operationsbereich angeordnet, sodass sie vom Operationspersonal manuell räumlich weitgehend frei bewegt und leicht positioniert werden können. Eine Aufnahmeoptik samt Videokameraeinheit ist zentrisch in der Operationsleuchte platziert und nimmt vom Ort der Operationsleuchte den gesamten Operationsbereich auf. Da der Abstand zwischen Operationsleuchte und Operationsbereich typischerweise im Bereich zwischen 1 und 3 m liegt, werden für die Aufnahmen von großformatigen Bildern vom Operationsbereich ebenso langbrennweitige Objektive mit den vorstehend genannten Nachteilen eingesetzt. Hinzu kommt, dass auch in diesem Fall der freie Sichtbereich zwischen Aufnahmeoptik und Operationsbereich durch die Köpfe der an der Operation beteiligten Personen oftmals verdeckt wird.

Mit allen bisher bekannten Aufzeichnungssystemen ist es nicht möglich, ein scharfes Abbild des unmittelbaren Operationsbereiches in Großaufnahme zu erhalten, ohne dabei den Arbeitsbereich für den Operateur einzuschränken.

US-A-4 854 301 offenbart eine Bildaufnahmevorrichtung gemäß dem Oberbegriff des Anspruchs 1.

### Darstellung der Erfindung

Der Erfindung liegt die Aufgabe zu Grunde, eine Bildaufnahmevorrichtung für den Einsatz im Sterilbereich zur visuellen Überwachung bzw. zur Dokumentation von einer, am menschlichen oder tierischen Körper durchzuführenden chirurgischen Operation innerhalb eines bestimmten Operationsbereiches, mit einer Kameraeinheit und einer mit dieser gekoppelten bildaufnehmenden Optikeinheit, die zusammen mit der Kameraeinheit extrakorporal derart positionierbar ist, dass die Optikeinheit einen Aufnahmebereich definiert, der zumindest den Operationsbereich enthält, derart weiterzubilden, dass ohne den Arbeitsbereich des Operateurs nachhaltig einzuschränken, die Bildaufnahmevorrichtung den Operationsbereich in Großbilddarstellung erfasst, sodass die vom Operateur innerhalb des Operationsbereiches unmittelbar vorgenommenen, zum Teil mikrochirurgischen Eingriffe exakt visuell erkennbar aufgenommen und zu Dokumentationszwecken sowie zu Studienzwecken auf entsprechenden Bildausgabemitteln optisch dargestellt werden können. Überdies soll die Bildaufnahmevorrichtung keine Probleme hinsichtlich Sterilisierbarkeit aufwerfen, sodass sie unmittelbar vom Operateur selbst im Sterilbereich hantiert werden kann.

Die Lösung der der Erfindung zu Grunde liegenden Aufgabe ist im Anspruch 1 angegeben. Den Erfindungsgedanken vorteilhaft weiterbildende Merkmale sind Gegenstand der Unteransprüche.

Erfindungsgemäß ist eine Bildaufnahmevorrichtung derart ausgebildet, dass die Optikeinheit in Art eines Sichtendoskops ausgebildet ist, das wenigstens einen optischen Übertragungskanal aufweist, dessen distales Ende extrakorporal, d.h. außerhalb des Körpers, gegenüber dem Operationsbereich räumlich positionierbar ist. Zur sicheren Handhabung des Sichtendoskops weist dieses, an seinem optischen Übertragungskanal eine mechanisch stabile Ummantelung auf, sodass der Operateur das Sichtendoskop an seinem Schaft zu Positionierzwecken entsprechend betätigen kann. Aus Gründen einer weitgehend freien Positionierbarkeit sowie einer Fixierung in einer gewünschten räumlichen Lage ist die Optikeinheit an ihrem proximalen Ende mit einer Positionier- und Haltevorrichtung verbindbar, vermittels der die Optikeinheit weitgehend frei verschwenkbar und räumlich fixierbar ist.

Der, der Erfindung zu Grunde liegende Gedanke betrifft die Verwendung eines an sich bekannten Sichtendoskops, jedoch nicht zur bildlichen Aufnahme von intrakorporal liegenden Körperbereiche, sondern vielmehr als Optikeinheit für die Bildaufnahme des Operationsbereiches aus einer Aufnahmeposition, die extrakorporal vom Operationsbereich beabstandet ist. Typische Abstände betragen wenige cm bis einige 10 cm. Im Unterschied zu konventionellen Sichtendoskopen, die einen optischen Übertragungskanal sowie wenigstens einen Beleuchtungskanal aufweisen, sieht die, in der erfindungsgemäß ausgebildeten Bildaufnahmevorrichtung verwendete Optikeinheit lediglich einen optischen Übertragungskanal vor, der von einer mechanisch stabilen Ummantelung umgeben ist. Durch die Ummantelung des optischen Übertragungskanals wird der Endoskopschaft stabilisiert, sodass der Operateur den Schaft unmittelbar ergreifen kann, um die Optikeinheit samt Kameraeinheit entsprechend zu verschwenken und zu positionieren.

Der besondere Vorteil bei der Verwendung einer Optikeinheit, die in Art eines Sichtendoskops ausgebildet ist, zur Aufnahme des Operationsbereiches liegt darin, dass sowohl der Sichtbereich als auch die Beleuchtungsverhältnisse durch das Sichtendoskop auf Grund seiner nur geringen räumlichen Dimensionen, nicht beeinträchtigt werden. Der Operateur kann trotz relativer Nähe des distalen Endes des Sichtendoskops am Operationsbereich frei und ungehindert seinem operativen Eingriff nachgehen und hat für die Operation optimale Sicht- und Beleuchtungsbedingungen, die auch in Gegenwart der Optikeinheit nicht beeinträchtigt werden.

Damit die Optikeinheit samt der mit ihr verbundenen Kameraeinheit leicht und nahezu widerstandsfrei vom Operateur selbst positioniert werden kann, ist das proximale Ende der Optikeinheit mit einem Stativarm verbunden, wobei die Verbindung zwischen Optikeinheit und Stativarm über eine Magnetschlussverbindung erfolgt. Der Stativarm selbst weist an seinem proximalen Ende einen sphärisch ausgebildeten Gelenkkörper auf, der in eine entsprechend an diesem angepasste sphärisch konkav ausgebildete Gegenkontur eines Haltemittels eingreift. Vorzugsweise ist der sphärische Gelenkkörper als Kugel ausgebildet, die über eine sphärische Kontaktfläche mit dem Haltemittel in Verbindung tritt, sodass die Kugel und das entsprechende Haltemittel gleitend zueinander gelagert sind. Aus Gründen einer selbsttätigen räumlichen Fixierung des Gelenkkörpers gegenüber dem Haltemittel sind im Haltemittel magnetkrafterzeugende Einheiten vorgesehen, beispielsweise in Form von Permanentmagneten oder Elektromagneten, die ein genügend großes Magnetfeld erzeugen, das in der Lage ist, den Stativarm samt Optik- und Kameraeinheit räumlich fest zu positionieren. Die zwischen dem sphärisch ausgebildeten Gelenkkörper, der vorzugsweise aus einem ferromagnetischen Material gefertigt ist und dem Haltemittel wirkenden Magnetkräfte sind dabei derart zu wählen, dass einerseits eine selbsttätige räumliche Fixierung der Optik- und Kameraeinheit in jeder konstruktiv vorgegebenen Lage möglich ist, jedoch andererseits die Magnetkräfte ein freies Verschwenken der Optik- und Kameraeinheit relativ zum Haltemittel durch bloßes Betätigen an der Ummantelung des Endoskopschaftes möglich ist.

In bevorzugter Weise kann aus Sterilitätsgründen das bei jeder Operation verwendete Operationstuch zwischen dem Haltemittel und dem sphärisch ausgebildeten Gelenkkörper eingebracht werden, sodass das Operationstuch auch für die gesamte Bildaufnahmevorrichtung als Trennmittel zwischen dem Steril- und Nicht-Sterilbereich dient. Alle oberhalb des Operationstuches befindlichen Komponenten der Bildaufnahmevorrichtung sind dabei derart ausgebildet, dass sie vollständig autoklavierbar sind. Zudem vermag das meist aus Textil- oder Baumwollstoff gefertigte Operationstuch als wünschenswertes Gleitmittel zwischen dem Haltemittel und dem sphärischen Gelenkkörper dienen.

Damit die Optik- und Kameraeinheit nebst Stativarm um den Gelenkkörper frei beweglich verschwenkbar ist und in einer gewünschten Raumlage fixiert bleibt, ist aus Balancegründen proximalseitig am Stativarm ein entsprechendes Gegengewicht anbringbar, das einen balancierenden Gewichtsausgleich zur distalen Last am Stativarm, bezogen auf den Bereich der Kontaktfläche am Gelenkkörper selbst, schafft. Das Gegengewicht kann in seiner Lage relativ zum Stativarm bewegt werden, sodass individuelle Hebelmomente als Ausgleichsmoment einstellbar sind.

Die vorstehend beschriebene Bildaufnahmevorrichtung vermag Großbildaufnahmen vom Operationsgeschehen am Operationsbereich aufnehmen, sodass sämtliche am Patienten durchzuführenden "Handgriffe" zu Dokumentationszwecken aufgenommen werden können. Der endoskopartig ausgebildete Schaft der Aufnahmeoptik ragt dabei von einer Seite in den extrakorporalen Arbeitsbereich des Operateurs, ohne diesen in seinem Betätigungsbereich zu beeinträchtigen. Durch die räumlich freie Positionierung der Optikeinheit kann der Operateur durch bloßes Betätigen am Endoskopschaft den Blickwinkel der Optikeinheit individuell verändern. Dies erfolgt ohne großen Kraftaufwand und ohne der Notwendigkeit eines Lösens bestimmter Haltegelenkfixierungen und entsprechendes Feststellen.

Da an sich bekannte Sichtoptiken von Sichtendoskopen auf Grund ihres optischen Aufbaus keinen begrenzten Fokuspunkt aufweisen, treten bei diesen Bildaufnahmen auch keine Unschärfeprobleme auf.

Durch einen bevorzugten Sichtwinkel von 30º bezogen auf die Endoskopschaftlängsachse kann das distale Ende der Optikeinheit seitlich im Operationsbereich positioniert werden und zugleich dafür sorgen, dass der interessante Operationsbereich vollständig optisch erfasst ist.

Da der Operationsbereich bereits durch die, an der Operation beteiligten Personen entsprechend eingestellten Operationsleuchten optimal ausgeleuchtet ist, benötigt die Bildaufnahmevorrichtung grundsätzlich keine zusätzlichen Lichtquellen. Bei besonders schwierigen Lichtverhältnissen kann jedoch in an sich bekannter Weise über die Ummantelung des optischen Übertragungskanals des Sichtendoskops ein zusätzlicher hohlkanalartig ausgebildeter Beleuchtungsendoskopschaft geschoben werden, der distalseitig zur Optikeinheit zusätzliches Beleuchtungslicht für die Bildaufnahme bereitstellt.

### Kurze Beschreibung der Erfindung

Die Erfindung wird nachstehend ohne Beschränkung des allgemeinen Erfindungsgedankens anhand eines Ausführungsbeispiels unter Bezugnahme auf die Zeichnung exemplarisch beschrieben. Es zeigt:

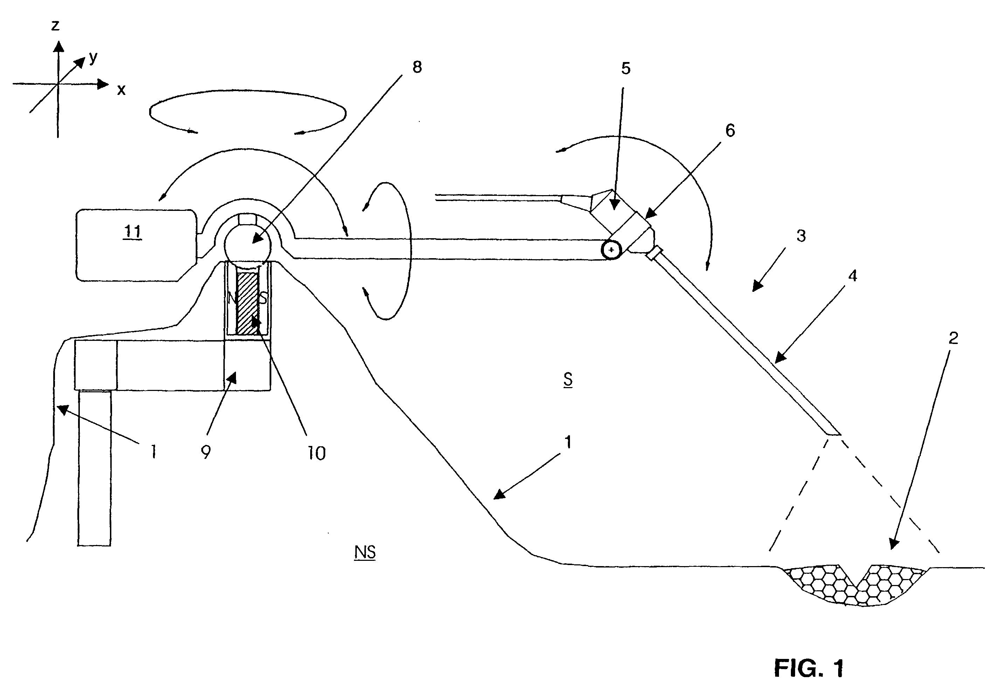

Fig. 1 schematisierte Darstellung einer Bildaufnahmevorrichtung.

### Wege zur Ausführung der Erfindung, gewerbliche Verwendbarkeit

Fig. 1 stellt stark schematisiert einen Querschnitt durch die Bildaufnahmevorrichtung dar, im Einsatz in einem Operationsraum. Das Operationstuch 1 trennt dabei den Sterilbereich S vom Nicht-Sterilbereich NS und weist überdies einen Ausschnitt auf, der den Operationsbereich 2 am Patienten freigibt. Den innerhalb des Operationsbereiches 2 stattfindenden Operationsvorgang gilt es nun mit der Bildaufnahmevorrichtung aufzuzeichnen. Hierzu weist die Bildaufnahmevorrichtung ein extrakorporal angebrachtes Sichtendoskop 3 auf, das distalseitig einen Bildaufnahmewinkel vorsieht, der gegenüber der Sichtendoskopschaftlängsachse um ca. 30º geneigt ist. Der Schaft 4 des Sichtendoskops 3 ist aus Gründen einer mechanischen Stabilisierung extra ummantelt, sodass der Operateur das Sichtendoskop an seinem Schaft 4 ergreifen und entsprechend positionieren kann. Die Positionierung erfolgt derart, dass das distale Ende des Schaftes 4 frei im Raum positionierbar ist, d.h. es muss nicht im Operationsbereich 2 gestützt oder gelagert werden. Proximalseitig ist das Sichtendoskop 3 mit einer Kamereraeinheit 5 verbunden, die die aufgenommenen Bildsequenzen über ein entsprechendes Kabel zur weiteren Bildverarbeitung weiterleitet. Kameraeinheit 5 und Sichtendoskop 3 sind ihrerseits vorzugsweise über eine magnetische Kupplung 6 oder über eine mechanisch arretierbare Kupplung mit einem Stativarm 7 verbunden, der proximalseitig einen sphärisch ausgebildeten Gelenkkörper 8 vorsieht, der in eine entsprechend ausgebildete Gegenkontur eines Haltemittels 9 eingreift. Zwischen dem sphärisch ausgebildeten Gelenkkörper 8 und dem Haltemittel 9 ist das Operationstuch 2 eingebracht, das zum einen als Trennung zwischen dem Steril- und Nicht-Sterilbereich dient, aber zum anderen eine wünschenswerte Gleitfunktion übernimmt, damit der sphärisch ausgebildete Gelenkkörper 8 innerhalb des Haltemittels 9 gleiten kann. Zur räumlichen Fixierung des Stativarms nebst daran angebrachter Optik- und Kameraeinheit dient ein Permanentmagnet 10, der im Haltemittel 8 integriert ist. Zum mechanischen Gewichtsausgleich ist zudem proximalseitig zum Stativarm 7 ein Gegengewicht 11 vorgesehen, das relativ zum Stativarm räumlich bewegt werden kann, um ein gewünschtes Gegenmoment zur distalen Last zu bilden.

Ferner ist anhand des eingezeichneten Koordinatensystem die freie Bewegbarkeit des Systems um die Raumachsen x, y und z zu entnehmen.

### Bezugszeichenliste

- 1: Operationstuch
- 2: Operationsbereich
- 3: Sichtendoskop
- 4: Schaft des Endoskops, optischer Übertragungskanal
- 5: Kameraeinheit
- 6: Magnetkupplung
- 7: Stativarm
- 8: Gelenkkörper
- 9: Haltemittel
- 10: Permanentmagnet
- 11: Gegengewicht
- S: Sterilbereich
- NS: Nichtsterilbereich

## Patentansprüche

1. Bildaufnahmevorrichtung für den Einsatz im Sterilbereich zur visuellen Überwachung und Dokumentation von einer, am menschlichen oder tierischen Körper durchzuführenden chirurgischen Operation innerhalb eines bestimmten Operationsbereiches, mit einer Kameraeinheit (5) und einer, mit dieser gekoppelten bildaufnehmenden Optikeinheit, die zusammen mit der Kameraeinheit extrakorporal derart frei positionierbar ist, dass die Optikeinheit einen Aufnahmebereich definiert, der zumindest den Operationsbereich enthält, wobei die Optikeinheit in Art eines Sichtendoskops (3) ausgebildet ist, das wenigstens einen optischen Übertragungskanal (4) aufweist, dessen distales Ende extrakorporal gegenüber dem Operationsbereich räumlich positionierbar ist, und wobei die Optikeinheit im Bereich ihres proximalen Endes mit einer als Magnetschlussverbindung (10) ausgebildeten Positionier- und Haltevorrichtung (7, 8) verbunden ist,
**dadurch gekennzeichnet, dass** der optische Übertragungskanal von einer Ummantelung umgeben ist, die derart mechanisch stabil ausgebildet ist, dass die Optikeinheit an der Ummantelung zu Positionierzwecken betätigbar ist, und dass vermittels der Positionier- und Haltevorrichtung (7, 8) die Optikeinheit weitgehend frei verschwenkbar und räumlich derart fixierbar ist, dass die Magnetkräfte gerade so groß gewählt sind, dass eine selbsttätige räumliche Fixierung der Optikeinheit in jeder konstruktiv vorgegebenen Lage möglich ist, und dass die Magnetkräfte so gering sind, dass ein Verschwenken der Optikeinheit durch Betätigen an der Ummantelung Gewährleistet ist.

2. Bildaufnahmevorrichtung nach Anspruch 1,
**dadurch gekennzeichnet, dass** die Optikeinheit einen Endoskopschaft (4) aufweist, der von der Ummantelung mechanisch verstärkt ist und einen Durchmesser aufweist, der weniger als 2 cm beträgt.

3. Bildaufnahmevorrichtung nach 2,
**dadurch gekennzeichnet, dass** das distale Ende der Optikeinheit derart ausgebildet ist, dass der Sichtwinkel relativ zur Endoskopschaftlängsachse zwischen 0° und 90°, vorzugsweise 30° beträgt.

4. Bildaufnahmevorrichtung nach einem der Ansprüche 1 bis 3,
**dadurch gekennzeichnet, dass** die Kameraeinheit unmittelbar ohne Zwischenoptik, wie bspw. eine Okularoptik, oder/und ohne Fokussiereinrichtung an das proximale Ende des Sichtendoskops ankoppelbar ist.

5. Bildaufnahmevorrichtung nach einem der Ansprüche 1 bis 4,
**dadurch gekennzeichnet, dass** die Positionier- und Haltevorrichtung wenigstens einen Stativarm (7) vorsieht, an dessen distalem Ende die Optikeinheit samt Kameraeinheit vorgesehen ist und an dessen proximalen Ende ein sphärischer Gelenkkörper (8) vorgesehen ist, der in eine entsprechend an diesem angepasste sphärisch konkav ausgebildete Gegenkontur eines Haltemittels eingreift, in der der Gelenkkörper nahezu ungehindert entlang der sphärischen Kontaktfläche zwischen Gelenkkörper und Haltemittel gleitend gelagert ist, und
dass zwischen Gelenkkörper und Haltemittel über die Kontaktfläche aus Gründen einer selbsttätigen räumlichen Fixierung des Stradivaris samt Optik- und Kameraeinheit Magnetkräfte wirken.

6. Bildaufnahmevorrichtung nach Anspruch 5,
**dadurch gekennzeichnet, dass** der sphärisch ausgebildete Gelenkkörper (8) aus ferromagnetischen Material besteht und dass in der Gegenkontur im Haltemittel ein Permanentmagnet (10) vorgesehen ist.

7. Bildaufnahmevorrichtung nach Anspruch 5 oder 6,
**dadurch gekennzeichnet, dass** entlang der Kontaktfläche zwischen dem Haltemittel und dem Gelenkkörper aus Sterilisationsgründen ein bei Operationen verwendbares Operationstuch (1) geführt ist, das den Sterilbereich vom Nichtsterilbereich abtrennt.

8. Bildaufnahmevorrichtung nach einem der Ansprüche 5 bis 7,
**dadurch gekennzeichnet, dass** proximalseitig am Stativarm ein Gegengewicht (11) anbringbar ist, das einen balanzierenden Gewichtsausgleich zur distalen Last am Stativarm bezogen auf den Bereich der Kontaktfläche schafft.

9. Bildaufnahmevorrichtung nach einem der Ansprüche 1 bis 8,
**dadurch gekennzeichnet, dass** um die Ummantelung des optischen Übertragungskanal ein hohlkanalartig ausgebildeter Beleuchtungsendoskopschaft anbringbar ist.

10. Bildaufnahmevorrichtung nach einem der Ansprüche 1 bis 9,
**dadurch gekennzeichnet, dass** das Sichtendoskop als stabförmige, optische Abbildungseinheit ausgebidet ist.

11. Bildaufnahmevorrichtung nach einem der Ansprüche 1 bis 10,
**dadurch gekennzeichnet, dass** die Kameraeinheit sowie die Optikeinheit als eine kompakte Baueinheit ausgebildet ist.

12. Bildaufnahmevorrichtung nach einem der Ansprüche 1 bis 12,
**dadurch gekennzeichnet, dass** die Positionier- und Haltevorrichtung (7, 8) derart ausgebildet ist, dass eine kontaktfreie Positionierung der Optikeinheit möglich ist.

## Claims

1. Video recording device for use in a sterile area for the visual observation and documentation of a surgical operation to be performed on the human or animal body within a defined operating zone, comprising a camera unit (5) and an optical unit for video recording, which is coupled to the camera unit and which can be freely positioned in an extra-corporeal position together with said camera unit in such a manner that said optical unit defines a recording zone that encloses at least said operating zone, with said optical unit being configured in the manner of a viewing endoscope (3) that includes at least one optical transfer passage (4) whose distal end can be positioned in a three-dimensional manner at an extra-corporeal position opposite to the operating zone, and wherein said optical unit is connected, in the zone of its proximal end, to a positioning and holding means (7, 8) configured in the form of a magnetic closing connector (10),
**characterised in that** said optical transfer passage is surrounded by an envelope formed with such a mechanical stability that said optical unit can be controlled on said envelope for positioning, and that said optical unit is largely freely pivotable and adapted to be fixed three-dimensionally in such a manner that the magnetic forces are chosen just so strong as to permit a three-dimensional automatic fixing of said optical unit in any structurally defined position, and that the magnetic forces are so weak that pivoting of said optical unit will be ensured by control of said envelope.

2. Video recording device according to Claim 1,
**characterised in that** said optical unit comprises an endoscope shaft (4) mechanically rigidified and presenting a diameter of less than 2 cm.

3. Video recording device according to Claim 2,
**characterised in that** the distal end of said optical unit is configured in such a way that the viewing angle is between 0° and 90°, preferably 30°, relative to the longitudinal axis of the endoscope shaft.

4. Video recording device according to any of the Claims 1 to 3,
**characterised in that** said camera unit can be directly connected, without an intermediate optical system, as an optical eyepiece system, for instance, or/and without focusing means, on the proximal end of the viewing endoscope.

5. Video recording device according to any of the Claims 1 to 4,
**characterised in that** said positioning and holding means is provided with at least one stand arm (7) on a distal end where said optical unit is arranged together with said camera unit, and with a proximal end where a spherical joint body (8) is disposed, which is engaged in a corresponding counter-contour of a holding means, which is adapted in a concave spherical shape to said joint, in which said joint body is engaged for sliding along the spherical contact surface, almost without any obstacle, between said joint body and said holding means, and
that for reasons of automatic three-dimensional fixing of the stand, including the optical unit and said camera unit, magnetic forces are effective between said joint body and said holding means.

6. Video recording device according to Claim 5,
**characterised in that** said spherically shaped joint body (8) is made of a ferromagnetic material, and that a permanent magnet (10) is arranged in the counter-contour in said holding means.

7. Video recording device according to Claim 5 or 6,
**characterised in that** an operation drape (1) is guided along the contact surface between said holding means and said joint body, which can be used for sterilisation purposes in surgical operations and which separates the sterile area from the non-sterile area.

8. Video recording device according to any of the Claims 5 to 7,
**characterised in that** a counter-weight (11) can be mounted, on the proximal side, to said stand arm, which creates a balancing equalisation of weight, relative to the distal load, on said stand arm, relative to the contact surface.

9. Video recording device according to any of the Claims 1 to 8,
**characterised in that** an illuminating endoscope shaft can be mounted around said envelope of said optical transfer, which presents the configuration of a hollow passage.

10. Video recording device according to any of the Claims 1 to 9,
**characterised in that** said viewing endoscope is configured in the form of an optical imaging unit in the form of a bar.

11. Video recording device according to any of the Claims 1 to 10,
**characterised in that** said camera unit as well as said optical unit is configured in the form of a compact structural unit.

12. Video recording device according to any of the Claims 1 to 11,
**characterised in that** said positioning and holding means (7, 8) is configured in such a way that it permits non-contact positioning of said optical unit.

## Revendications

1. Dispositif d'enregistrement vidéo pour l'emploi dans une zone stérile pour le monitorage visuel et la documentation d'une opération chirurgicale à réaliser sur le corps humain ou animal à l'intérieur d'une zone d'opération définie, comprenant une unité de caméra (5) et une unité optique d'enregistrement vidéo accouplée à la dernière, qui est apte à être librement positionnée en une position extra-corporelle ensemble avec ladite unité de caméra d'une telle manière, que ladite unité optique définit une zone d'enregistrement, qui renferme au moins ladite zone d'opération, à ladite unité optique étant configurée de la façon d'un endoscope de vue (3), qui comprend au moins un canal de transfert optique (4), dont l'extrémité distale est apte à être positionnée de manière tridimensionnelle en une position extra-corporelle en face de la zone d'opération, et dans lequel ladite unité optique est reliée, dans la zone de son extrémité proximale, à un dispositif de positionnement et de tenue (7, 8) configuré sous forme d'un raccord de fermeture magnétique (10),
**caractérisé en ce que** ledit canal de transfert est entouré par une chemise formée à une telle stabilité mécanique, que ladite unité optique soit commandable à ladite chemise aux fins de positionnement, et **en ce que** ladite unité optique est largement librement pivotable et apte à être fixée à trois dimensions d'une telle manière, que les forces magnétiques soient choisies juste si grandes, qu'une fixation tridimensionnelle automatique de ladite unité optique soit possible en toute position définie par construction, et **en ce que** les forces magnétiques soient si petites, qu'un pivotement de ladite unité optique soit assuré par commande à ladite chemise.

2. Dispositif d'enregistrement vidéo selon la revendication 1,
**caractérisé en ce que** ladite unité optique comprend une tige d'endoscope (4) rigidifiée, de façon mécanique, par ladite chemise et présentant un diamètre de moins de 2 cm.

3. Dispositif d'enregistrement vidéo selon la revendication 2,
**caractérisé en ce que** l'extrémité distale de ladite unité optique est configurée d'une telle façon, que l'angle de vue soit entre 0° et 90°, de préférence 30°, relativement à l'axe longitudinal de la tige d'endoscope.

4. Dispositif d'enregistrement vidéo selon une quelconque des revendications 1 à 3,
**caractérisé en ce que** ladite unité de caméra est apte à être raccordée directement, sans système optique intermédiaire comme un système optique d'oculaire, par exemple, oulet sans moyen de focalisation, à l'extrémité proximale de l'endoscope de vue.

5. Dispositif d'enregistrement vidéo selon une quelconque des revendications 1 à 4,
**caractérisé en ce que** ledit moyen de positionnement et de tenue est muni d'au moins un bras de statif (7) à une extrémité distale, où ladite unité optique est disposée ensemble avec ladite unité de caméra, et à une extrémité proximale, où un corps de joint sphérique (8) est disposé, qui se trouve en prise dans un contre-profil correspondant d'un moyen de tenue, qui est adapté, sous forme sphérique concave, audit joint, dans lequel s'appuie ledit corps de joint à glissement le long de l'aire de contact sphérique, presque sans obstacle, entre ledit corps de joint et ledit moyen de tenue, et
**en ce que** pour des raisons d'une fixation automatique à trois dimensions du statif, y compris l'unité optique et ladite unité de caméra, des forces magnétiques sont effectives entre ledit corps de joint et ledit moyen de tenue.

6. Dispositif d'enregistrement vidéo selon la revendication 5,
**caractérisé en ce que** ledit corps de joint (8) à configuration sphérique est fait en un matériau ferromagnétique, et **en ce qu'**un aimant permanent (10) est disposé dans le contre-contour dans ledit moyen de tenue.

7. Dispositif d'enregistrement vidéo selon la revendication 5 ou 6,
**caractérisé en ce qu'**un tissu chirurgical (1) est guidé le long de l'aire de contact entre ledit moyen de tenue et ledit corps de joint, qui est utilisable aux fins de stérilisation aux opérations chirurgicales et qui sépare la zone stérile de la zone non stérile.

8. Dispositif d'enregistrement vidéo selon une quelconque des revendications 5 à 7,
**caractérisé en ce qu'**un contre-poids (11) est apte à être monté, du côté proximal, audit bras de statif, qui engendre une égalisation de poids de balance, relativement à la charge distale, audit bras de statif, relativement à la zone de l'aire de contact.

9. Dispositif d'enregistrement vidéo selon une quelconque des revendications 1 à 8,
**caractérisé en ce qu'**une tige d'endoscope d'illumination est apte à être monté autour de ladite chemise dudit canal de transfert optique, qui a la configuration d'un passage creux.

10. Dispositif d'enregistrement vidéo selon une quelconque des revendications 1 à 9,
**caractérisé en ce que** ledit endoscope de vue est configuré sous forme d'une unité d'image optique sous forme de barre.

11. Dispositif d'enregistrement vidéo selon une quelconque des revendications 1 à 10,
**caractérisé en ce que** ladite unité de caméra ainsi que ladite unité optique est configurée sous forme d'une unité structurelle compacte.

12. Dispositif d'enregistrement vidéo selon une quelconque des revendications 1 à 11,
**caractérisé en ce que** ledit moyen de positionnement et de tenue (7, 8) est configuré d'une manière à permettre un positionnement sans contact de ladite unité optique.
